# EUROPEAN PATENT SPECIFICATION

(11) **EP 1 656 089 B1**
(45) Date of publication and mention of the grant of the patent: **22.06.2011**
(21) Application number: 04756686.4
(22) Date of filing: 06.07.2004
(51) Int. Cl.: A61F 13/15

(54) **EFFICIENTLY MANUFACTURABLE ABSORBENT DISPOSABLE UNDERGARMENT AND METHOD OF MANUFACTURING ABSORBENT DISPOSABLE ARTICLE**
EFFIZIENT HERSTELLBARES ABSORBIERENDES WEGWERFHÖSCHEN UND HERSTELLMETHODE FÜR ABSORBIERENDE WEGWERFARTIKEL
SOUS-VETEMENT ABSORBANT JETABLE FABRIQUE DE FA ON EFFICACE ET PROCEDE DE FABRICATION D'ARTICLES ABSORBANTS JETABLES

(30) Priority: 10.07.2003 US 617218
(43) Date of publication of application: 17.05.2006
(73) Proprietor: First Quality Retail Services, LLC, Great Neck, NY 11021 (US)
(72) Inventor: GLAUG, Frank, S., Chester Springs, PA 19425 (US); HOOD, Prelo, M., Schwenksville, PA 19473 (US); RICE, Jocelyn, P., Philadelphia, PA 19151 (US)
(74) Representative: Moore, Christopher Mark
(86) International application number: PCT/US2004/021602
(87) International publication number: WO 2005/007051

(56) References cited:
- EP-A- 0 048 011
- EP-A- 1 155 668
- EP-A- 1 240 881
- EP-A- 1 249 214
- WO-A-2004/060252
- US-A1- 2003 217 447
- US-B1- 6 514 233

## Description

### FIELD OF THE INTENTION

This invention relates generally to disposable absorbent undergarments, e.g., disposable underwear or briefs adults and youth and training pants for children, and more specifically to articles of that type that can be manufactured very efficiently and economically and which exhibit good protection from leakage, good fit and comfort.

### BACKGROUND OF THE INVENTION

Disposable absorbent articles, such as children's training pants, youth pants and adult incontinent briefs or underwear, are designed to absorb and contain body waste, e.g., urine and/or feces, to prevent such waste from soiling, wetting, or otherwise contaminating clothing or other articles, such as bedding, that come in contact with the wearer. Many such articles are commercially available. Moreover, the patent literature is replete with examples of various pant or brief constructions and methods of manufacturing them.

For example, In United States Letters Patent No: Patent No. 4,764,234 (Smits, et al.), which is assigned to the same assignee as this invention, there is disclosed a method of manufacturing an absorbent pad for utilization as a diaper or an adult incontinent brief. The method entails providing a moving web of material forming sequential backing sheets for the article. Nozzles are utilized to apply a pair of continuous bands of adhesive to the back sheet of the pad, prior to its assembly. The nozzles eject the adhesive onto a moving web which makes up the backing sheet and are shifted sideways during their extrusion of adhesive from a first location to a second location. This action generates a pair of nonlinear bands of adhesive on the backing sheet. The nozzles are again shifted from their second location to their original first location. The elastic band is not shifted when the nozzles are, thus effectuating securement of the elastic bands only on the portions of the backing sheets where the adhesive was applied at the first location. The sequentially located sheets are cut on opposite marginal side to form leg cut-out areas, whereupon only those portions of the elastic bands at the center of each of the leg cut-out areas contract to form a gather thereat.

United States Letters Patent No. 4,764,242 (Gressick et al.), which is also assigned to the same assignee as this invention, discloses a machine for manufacturing a diaper or brief fike that disclosed in the aforementioned Smits et al. patent.

United States Letters Patent No. 4,795,451 (Buckley), which is also assigned to the same assignee as this invention, discloses a disposable absorbent pad for use as a baby diaper or an adult incontinent brief wherein the backing sheet of the diaper has a pair of continuous bands of adhesive arranged in a stepped generally longitudinal configuration thereon. An elastic band is disposed over the mid-portion of the adhesive being stepped to the side, out of contact with the elastic. When the pads are cut in the assembly process, only that portion of elastic in contact with the adhesive causes the pad to gather. The remaining end portions of the adhesive bands are secured to the intermediate layer of absorbent fluff material, helping it remain in place, and act as a fluid barrier on the sides of the absorbent pad.

United States Letters Patent 4,804,379 (Toth et al.). which is also assigned to the same assignee as this invention discloses a disposable absorbent pad for utilization as a diaper or adult brief. The pad is an absorbent member covered on one side by a sheet of fluid impervious material, and on the other side by a pair of generally parallel strips of fluid impervious material overlapping the absorbent pad and the backing sheet, in the crotch area to prevent leakage of fluid from the absorbent pad in the crotch area. The pad includes a pair of opposed leg cut-outs each of which is elasticized by a linear elastic band.

In United States Letters Patent 5,649,919 (Roessler et al.), there is disclosed an absorbent article which has a lateral width, a longitudinal length, longitudinally extending side margins, a front waistband portion, a back waistband portion, and an intermediate portion which interconnects the front and back waistband portions. The article comprises a backsheet layer and an absorbent retention portion superposed on the backsheet layer. A liquid permeable topsheet layer is superposed on the retention portion to sandwich the retention portion between the topsheet layer and the backsheet layer. An elasticizing means form elasticized gathers at leg opening portions of the article. The elasticizing means include a front set of laterally opposed, longitudinally extending leg elastic members located in the article side margins in at least the intermediate portion of the article. The front elastic members are arranged asymmetrically with respect to the article length and have a selected offset toward the front waistband portion of the article. A back set of laterally opposed, longitudinally extending leg elastic members are constructed separate from the front set of elastic members and are located in the article side margins in at least the intermediate portion of the article. The back elastic members are arranged asymmetrically with respect to the article length and have a selected offset toward the back waistband portion of the article.

In United States Letters Patent 5,904,675 (Laux et al.), there is disclosed an absorbent article has a longitudinal length dimension, a lateral cross dimension, a front waistband portion, a back waistband portion and an intermediate portion which interconnects the front and back waistband portions. The article includes a backsheet layer having a pair of laterally opposed side margins, with each side margin having an outwardly concave, terminal side edge contour located at appointed leg opening regions in an intermediate portion of each of the side margins. Each concave side edge contour has a selected longitudinal extent along the length dimension of the article. A liquid permeable topsheet layer is connected in superposed relation to the backsheet layer, and an absorbent body is sandwiched between the topsheet layer and the backsheet layer. A separate, elasticized and gathered leg gusset is connected to the article along each of the appointed leg opening regions, and each leg gusset is configured to extend beyond and to bridge between opposed, spaced-apart portions of an associated one of the concave side edge contours of the backsheet layer.

In United States Letters Patent 5,906,603 (Roe et al.), there is disclosed a disposable absorbent article including a topsheet, a backsheet, and an absorbent core having a pair of opposed longitudinal edges. The absorbent article includes a leg cuff extending at least laterally outwardly from each opposed longitudinal edge. Each leg cuff has a proximal edge, a distal edge and an elasticized region disposed between the proximal edge and the distal edge. The elasticized region has an inner edge, an outer edge and a width. The proximal edge of each leg cuff extends generally longitudinally along and adjacent to at least a portion of one of the opposed longitudinal edges and the distal edge of each of said leg cuffs is disposed laterally outwardly from the proximal edge. Each leg cuff includes an inner bond disposed adjacent at least a portion of the proximal edge of each leg cuff. An outer bond is spaced laterally outwardly from the inner bond, the spacing between the inner bond and said outer bond defining a leg cuff base width. Each leg cuff also includes an innerwall that extends upwardly and laterally outwardly from the inner bond to the inner edge of the elasticized region having a first height, and an outer wall that extends upwardly and laterally inwardly from the outer bond to the outer edge of the elasticized region having a second height. An elastic element is disposed in the elasticized region of each leg cuff and is provided generally parallel to the wearer's skin.

In United States Letters Patent 6,010,586 (Suprise), there is disclosed a disposable absorbent article which defines a first side portion, a second side portion, and a longitudinal centerline between the side portions. The absorbent article comprises an outer cover and an absorbent insert which is connected to the outer cover. The outer cover comprises a first side panel which is located in the first side portion of the absorbent article and a second side panel which is located in the second side portion of the absorbent article. An edge of the first side panel is connected to an edge of the second side panel to provide a seam which extends along the longitudinal centerline between the side portions of the absorbent article. The opposite waist regions on each side panel are configured to encircle the legs of the wearer and releasably engage together about the hips of the wearer., The disposable absorbent article has an aesthetically pleasing garment-like appearance and is readily refastenable about the hips of the wearer.

In United States Letters Patent 6,217,563 (Van Gompel et al.) there is disclosed an integral absorbent article, such as diaper. The diaper has a longitudinal article length and a lateral article width and includes an absorbent composite having first and second longitudinally opposed end regions, and at least a first longitudinally terminal end edge. The absorbent composite includes a substantially liquid-impermeable backsheet layer, a substantially liquid permeable topsheet layer, and a retention portion sandwiched between the backsheet and topsheet layers. The retention portion has laterally opposed, terminal side edges, and the absorbent composite includes a pair of laterally opposed side margins which extend laterally beyond the side edges of the retention portion. A first body panel is joined to the first longitudinal end of the absorbent composite. The first body panel has a body side surface, an outward surface, a panel length which is less than the article length, an outboard terminal end edge, and a relatively inboard terminal end edge. Each of the side margins of the absorbent composite is inwardly turned to provide a turned bodyside surface and a turned outward side surface of each side margin. At least a first portion of the turned bodyside surface of each side margin of the absorbent composite is secured to the outward surface of the first body panel with a body panel attachment.

In International Patent Application WO 00/37010 (Roessler et al.) there is disclosed a pant-like, refastenable, disposable absorbent article that includes an absorbent chassis and a pair of opposed side panels. The absorbent chassis defines a pair of laterally opposed side edges (30) and a pair of longitudinally opposed waist edges. The side panels extend between the side edges of the absorbent chassis to define a waist opening and a pair of leg openings in the pant-like disposable absorbent article. Each of the opposed side panels defines a first side margin which is permanently attached to the side edge (30) of the absorbent chassis in a back waist region of the absorbent article to provide a permanent joint. Each of the opposed side panels further defines a second side margin opposite the first side margin which is refastenably attached to the side edge of the absorbent chassis in the front waist region of the absorbent article to provide a refastenable joint. This application also discloses the process of making the article. In particular, the process entails providing a continuous web of interconnected absorbent chasses. A pair of laterally opposed back panels are attached to the side edges of each of the absorbent chasses in one of the front waist region or the back waist region to provide a permanent joint. A pair of laterally opposed front panels are refastenably attached to the side edges of each of the absorbent chases in the opposite waist region of the absorbent article to provide a refastenable joint. The continuous web of interconnected absorbent chasses are cut into discrete absorbent articles and are folded about a fold line extending in a lateral direction through the crotch region of the article, thereby positioning the front panels and back panels in a facing relationship. The front and back panels are fastened together along a pair of laterally opposed side seams to define a waist opening and a pair of leg openings for the pant-like, refastenable, disposable absorbent article.

In United States Letters Patent Number 6,478,786 (Glaug), which is assigned to the same assignee as this invention, there is disclosed an absorbent article, e.g., a diaper, having a crotch portion, a waist portion, a front belly portion and a rear back portion. The waist, belly and back portions include plural transversely oriented elastic threads. The diaper includes an absorbent core. Longitudinally oriented elastic threads are located on opposite sides of the core and intersect the transverse elastic threads to enclose the core. The diaper includes a pair of leg openings, each of which has a first arcuate section, a second arcuate section, and an intermediate section. The first arcuate section includes at least one arcuate elastic thread, as does the second arcuate section. Those threads are spaced by a gap. A portion of the longitudinally oriented elastic threads on either side of the core is located closely adjacent a respective gap between the arcuate elastic threads to elasticize the leg openings.

In United States Letters Patent Number 6,514,233 (Glaug et al.), which is assigned to the same assignee as this invention, there is disclosed a disposable absorbent article, e.g., a diaper, and a method of manufacturing plural diapers. Each diaper includes a front section, a rear section and an intermediate section. Each section is a generally planar member formed of a flexible material having a top edge, a bottom edge and an opposed pair of side edges. The bottom edge of the front section is somewhat concave, and the bottom edge of the rear section is somewhat convex. The intermediate section is formed of a fluid pervious cover-stock, a fluid impervious barrier layer, and an absorbent core interposed therebetween. The intermediate section is of generally rectangular shape having a pair of elasticized sides and an pair of end portion one of which is adhesively secured to the middle of the front section, while the other end portion is similarly secured to the middle of the rear section. The front and rear sections are arranged to be releasably secured to each other by fastening tapes to mount the diaper. A portion of the bottom edge of the front section, one side of the intermediate section and a corresponding portion of the bottom edge of the rear section form one leg hole of the diaper. Corresponding portions of the front, rear and intermediate sections form the other leg hole. The tensioned intermediate section forms a pair of upstanding walls conforming to the crotch of the wearer to prevent leakage from the diaper. The diapers are made by providing plural sheet units, each making up a front section of one diaper and a rear section of the next succeeding diaper. Those units are adhesively secured to respective ends of an intermediate section in a sequential line of such sections. Then the two sheet units that are secured to the intermediate section are each severed transversely to complete a diaper. This process is repeated for succeeding diapers.

While the prior art disposable absorbent diapers and briefs may be generally suitable for their intended purposes, they still leave much to be desired from the standpoints simplicity of construction and of ease and economy of manufacture. Thus, a need presently exists for a child's training pants, a youth's pants and an adult brief or underwear that is simple in construction, can be manufactured economically and efficiently without wastage, and which provides a good fit, resistance to leakage and wearing comfort. It is to those ends that this invention is directed.

### SUMMARY OF THE INVENTION

One aspect of this invention is directed to disposable absorbent undergarment, e.g., a child's training pants, a youth's pants or an adult brief, arranged to be worn by a wearer to trap and collect waste products. Another aspect of this invention is directed to a method of economically manufacturing a disposable absorbent article, such as a diaper or an undergarment.

The undergarment basically comprises a pants-shaped chassis having a front waist portion, a belly portion, a rear waist portion, a rear back portion, a crotch portion, and a pair of leg openings disposed on opposite sides of the crotch portion. The crotch portion is located between the leg openings and has a front section and a rear section. The front waist portion and the rear waist portion are joined together at the sides of the chassis. The belly portion and the rear back portion are also joined together at the sides of the chassis.

Each of the leg openings has a high-cut concave edge portion at the front section, and a convex edge portion at the rear section. The concave edge portion of one of the leg openings is of complementary shape to the convex edge portion of the one of the leg openings. The concave edge portion of the other of the leg openings is of complementary shape to the convex edge portion of the other of the leg openings.

With this construction when the undergarment is worn it covers a substantial portion of the wearers leg beneath the buttocks, while a substantial portion of the wearer's leg where the leg meets the torso is exposed.

The method aspect of this invention entails manufacturing plural disposable absorbent articles, e.g., undergarments, like those described above, or diapers, in an economical manner. The method basically comprises the steps of providing a web of material having a first longitudinal axis, a pair of generally linear side edges, a first elongated elastic member extending generally linearly parallel to the first longitudinal axis adjacent one of the side edges, a second elongated elastic member extending generally linearly parallel to the first longitudinal axis adjacent the other of the side edges, at least a third undulating curved elastic member extending generally parallel to the first longitudinal axis adjacent the first elastic member, and at least a fourth undulating curved elastic member extending generally parallel to the first longitudinal axis adjacent the second elastic member. The web is severed along a longitudinal line between the third and fourth elastic members and generally parallel to the first longitudinal axis to form a front web section and a rear web section. The front and rear web sections are separated from each other. An intermediate section is provided having a second longitudinal axis, a pair of marginal side edges extending generally parallel to the second longitudinal axis, a front edge portion, a rear edge portion, a first elongated elastic member extending generally parallel to one of the marginal side edges, and a second elongated elastic member extending generally parallel to the other of the marginal side edges. The front edge portion of the intermediate section is secured to the front web section and the rear edge portion of the intermediate section is secured to the rear web sections so that the second longitudinal axis is generally perpendicular to the first longitudinal axis. The front and rear web sections are severed along respective transverse lines extending perpendicular to the first longitudinal axis on opposite sides of the insert section.

If the article is an undergarment the method also entails folding the intermediate section to bring portions of the front and rear sections contiguous with the transverse lines into engagement with each other and securing those sections together, so that the severed front web section forms a front portion of the undergarment and the severed rear web section forms a rear portion of the undergarment.

### DESCRIPTION OF THE DRAWING

Fig. 1 is a front view of one exemplary embodiment of a protective undergarment constructed in accordance with this invention;
Fig. 2 is a rear view of the protective undergarment shown in Fig. 1;
Fig. 3 is an illustration of one initial step in the manufacturing process for making the protective undergarment shown in Figs. 1 and 2, wherein were the two webs of material are adhesively secured with the elastic threads interposed between the webs;
Fig. 4 is an illustration showing the assembled web at a further step in the manufacturing process for making the protective undergarment shown in Figs. 1 and 2, wherein the assembled web is are shown prior to being severed to form a front section web and a rear section web;
Fig. 5 is an illustration, like Fig. 4, but showing the line along which the web is severed to form the front and rear web sections;
Fig. 6 is an illustration, like Figs. 4 and 5, but showing the web at a later point in time after it has been severed into the front and rear web sections;
Fig. 7 is an illustration, like Figs. 4-6, but showing the web sections at a still later point in time wherein an intermediate section is registered thereon to form the crotch portion of the undergarment;
Fig. 8 is an illustration, like Figs. 4-7, but showing the web sections at a still later point in time wherein the assembled item has been folded at its intermediate section so that the front web section and the rear web sections overlie each other;
Fig. 9 is an illustration, like Figs. 4-8, but showing the web sections at a still later point in time wherein the folded assembly has been sealed at respective sides of one of the items the undergarment, with the upstream most item of undergarment shown being severed from the line of the remaining items to complete one item of undergarment; and
Fig. 10 is an enlarged sectional view taken along line 10 - 10 of Fig. 7

### DETAILED DESCRIPTION OF THE PREFERRED EMBODIMENT

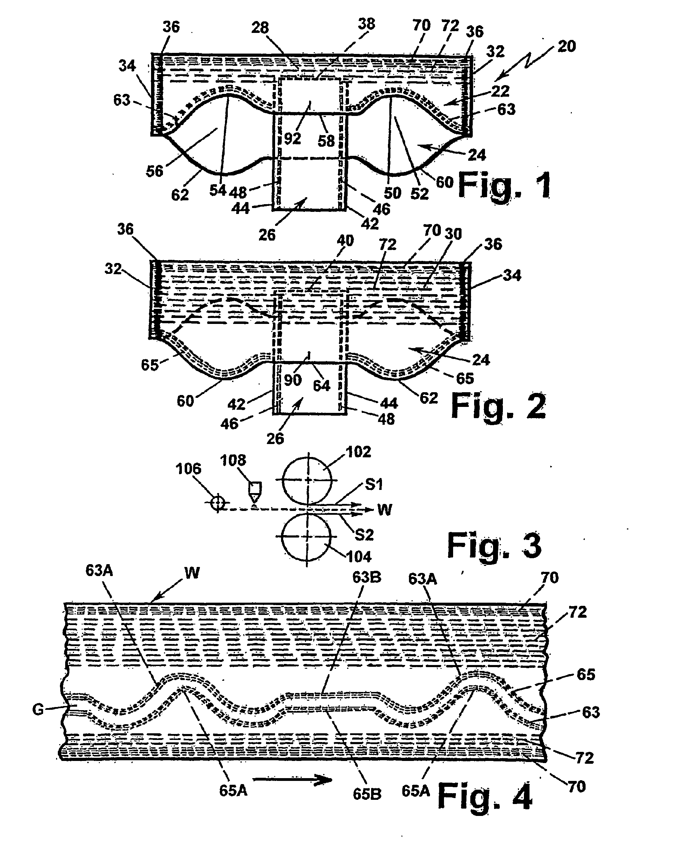

Referring now to the various figures of the drawing wherein like reference characters refer to like parts, there is shown in Figs. 1 and 2 a disposable absorbent article 20 in the form of a protective undergarment, such as a child's training pants, a youth's pants or an adult brief, that is constructed in accordance with one exemplary embodiment of this invention. It should be pointed out that as used herein the term "undergarment" means an article that is in the form of a pants or brief to enable it to be readily pulled on or pulled off, like conventional underwear. Moreover, the term "disposable" means that the undergarment is designed to be used until soiled, either by urination or otherwise, and then discarded, rather than being washed and used again. The undergarment 20 is formed of the same basic materials as that used to form the undergarment of United States Letters Patent No. 6,478,786.

To that end the undergarment 20 comprises a chassis including front portion or section 22, a back portion or section 24 (Fig. 2), and part of an Intermediate section 26 that forms the crotch portion of the undergarment. As can be seen in Fig. 1, the front section 22 includes a waist and adjacent belly area 28 Including plural transversely extending elastic threads (to be described later). The rear section 24 also includes a waist and adjacent lower back area 30 including plural transversely extending elastic threads 30 (also to be described later). The front section 22 and the rear section 24 are joined together at their respective side edges 32 and 34 by seals 36 extending along those side edges. The seals can be formed by any suitable means or technique, e.g., heat sealing, ultrasonic sealing, adhesive sealing, etc.

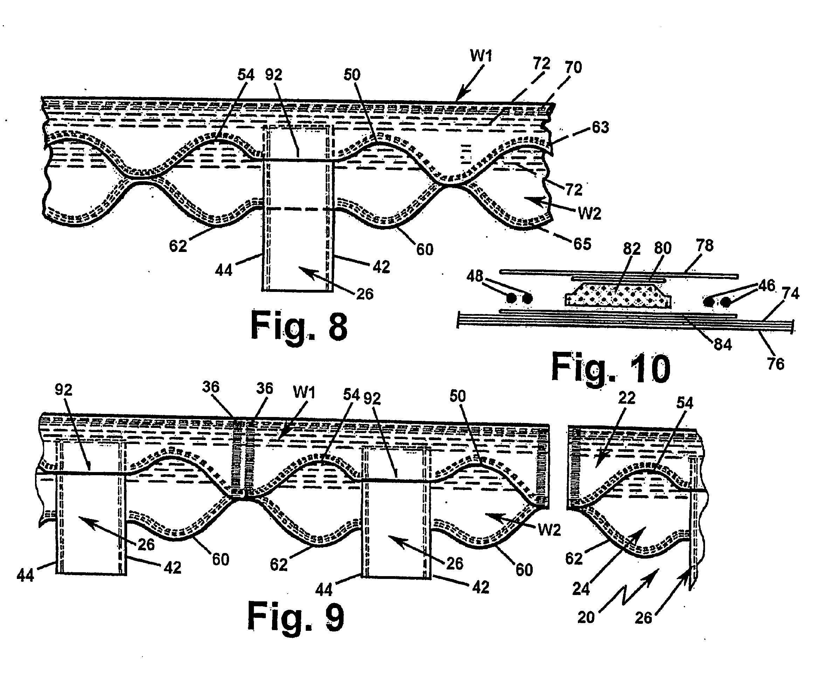

The intermediate section 26 will be described later with reference to Fig. 10. Suffice it for now to state that the section 26 is a generally planar, rectangularly shaped member having a front top edge 38 (Fig. 1) a back or rear top edge 40 (Fig. 2) and an opposed pair of linear side edges 42 and 44. A pair of elastic threads 48 are adhesively secured to the section 26 along its side marginaledge42 and another pair of elastic threads 48 are adhesively secured to the section 26 along its side marginal edge 44. As will be descnbed later, the threads 46 and 48 are pretensioned, i.e., secured to the section while under tension. The intermediate section 26 is adhesively secured to the inner surface of the front section 22 by an adhesive located in the area contiguous with the front top edge 38. In a similar manner, the intermediate section is adhesively secured to the inner surface of the back section 24 by an adhesive located in the area contiguous with the back top edge 40.

The lower edge of the front section 22 includes a portion which is somewhat s-shaped and is contiguous with the side 32. The S-shaped portion includes a concave recess 50. This recess forms a high-cut upper front portion of one of the undergarment's leg openings. That opening is designated by the reference number 52. In a similar manner a mirror image S-shaped arcuate r portion is provided in the lower edge of the front section 22 contiguous with the side 34. This arcuate portion includes a concave recess 54 that forms a high-cut upper front portion of the other of the garment's leg openings 56. The remainder of the lower edge of the front section is a linear portion 58 located between the recesses 50 and 54.

The lower edge of the rear or back section 24 includes a portion which is somewhat S-shaped and is contiguous with the side 34. This arcuate portion includes a convex projection 60 forming a lower rear portion of the undergarment's leg opening 52. In a similar manner a mirror image S-shaped arcuate portion is provided in the lower edge of the back section 24 contiguous with the side 34. This arcuate portion includes a convex projection 62 forming a lower rear portion of the undergarment's leg opening 56. The remainder of the lower edge of the rear section 24 is a linear portion 64 located between the projections 60 and 62.

Plural pretensioned elastic fibers 63 are adhesively secured along the arcuate portions of the bottom edge of the front section 22 and plural pretensioned elastic fibers 65 are adhesively secured along the arcuate portions of the bottom edge of the rear section 24. These fibers extend parallel to each other and follow the shape of the adjacent lower edge of the garment's front or rear section at which they are located.

With the intermediate section 26 secured to the front and rear sections 22 and 24, respectively, the leg opening 52 is established by the concave recess 50 of the front section 22, the contiguous linear edge 42 of the intermediate section 26 and the convex projection 60 of the rear section 24. In a similar manner the other leg opening 56 of the undergarment is established by the concave recess 54 of the front section 22, the contiguous linear edge 44 of the intermediate section 26 and the lower projection 62 of the rear section 24. Each of these leg openings is elasticized by the elastic fibers located adjacent the various portions of each of those openings.

As will be appreciated by those skilled in the art, the elasticized leg openings 52 and 56 exhibit a high-cut front portion to ergonomically accommodate the portion of the wearer's leg that merges with his/her torso. This enables the wearer to comfortably sit or stand while wearing the garment, yet provides a sufficiently tight seal to preclude the egress of liquid out through the leg opening. The projections 62 and 64 of the leg openings serve to cover a substantial portion of the wearer's body where his/her buttocks merge with the upper leg, thereby providing very good coverage of the buttocks to collect and retain waste produced by the wearer.

As will be discussed later, the front and rear sections 22 and 24, respectively, are created at the same time from a single web (of either single ply or layer or multiple plies or layers) that is severed longitudinally along a single line, with the line forming the lower edge of the front section and the lower edge of the rear section. Thus, the two sections are of complementary shape.

As can be seen in Fig. 3, the front and rear sections of the chassis are composed of a pair of continuous webs or sheets S1 and S2 and are joined to produce a web W (to be described later). This web serves to form the "backsheet" of the undergarment. In this exemplary embodiment, the back sheet is a two-ply member formed of a cloth-like, non-woven, breathable material (to be described later).

As will be appreciated from the discussion to follow, the undergarment 20 is elasticized by the transversely extending elastics mentioned earlier to enable it to be readily pulled on and pulled off, and when in place to conforms to the body of the wearer, while also precluding the egress of any liquid such as urine or loose feces from it. To that end, the front and rear sections of the chassis are elasticized by the use of a plurality of elastic fibers or filaments which are glued in place between two webs of material S1 and S2 used to form a portion of the undergarments. In particular, as best seen in Fig. 2, a first group or plurality of elastic filaments or fibers 70 are included in the chassis and extend transversely across the chassis from side 32 to side 30 of the front section 22 immediately adjacent its top edge. The portion of the front section 22 contiguous with the top edge will be referred to as the front waist section. The portion of the front section below the front waist section will be referred to as the front belly section. The portion of the rear section 24 contiguous with the top edge will be referred to as the rear waist section and the portion of the rear section below it will be referred to as the rear back section. A similar group of elastic fibers 70 are included in a similar manner in the rear waist section (Fig. 2).

The transversely oriented elastic fibers 70 of both the front and rear waist sections are closely spaced parallel to one another, e.g., 6.35mm (0.25 inch). A second group or plurality of identical elastic fibers 72 are also included in the chassis and extend across the chassis from side 32 to side 34 in the front belly section and In the rear back section. The transversely oriented, second group of fibers 72 in each such group are also equidistantly spaced from one another, but at a greater spacing than the fibers 70 of the first groups, e.g., at 12.7mm (0.5 inch) spacing.

As mentioned earlier, the fibers 63, 65, 70 and 72 are included in the chassis under tension (they are stretched) and as shown in Fig. 3 are placed between the inner surfaces of the two webs S1 and S2. Those webs are provided from rolls 102 and 104. The elastic threads making up the fibers 63, 65, 70 and 72 are provided from a roll or rolls 106 and are stretched. An applicator 108 provides a suitable adhesive to secure the fibers in place between the webs S1 and S2. The two webs with the Interposed stretched fibers are adhesively secured to each other by any suitable adhesive to complete the two-ply web W of material from which the front and rear sections 22 and 24, respectively, are formed.

The material forming the front and rear section of the chassis can be any suitable material used in disposable absorbent articles. In the interest of wearing comfort, it is preferably a non-woven, cloth-like breathable material, such as spunbond/meltblown/spunbond polypropylene at 15g/m² (15 gsm) available from Avgol of Holon, Israel. The material making up the chassis may be a polylaminate, e.g., polyfilm bonded to a non-woven material. The adhesive for securing the webs S1 and S2 together with the elastic fibers interposed therebetween, may be a construction adhesive, such as that available from National Starch and Chemical Company of Bridgewater, New Jersey.

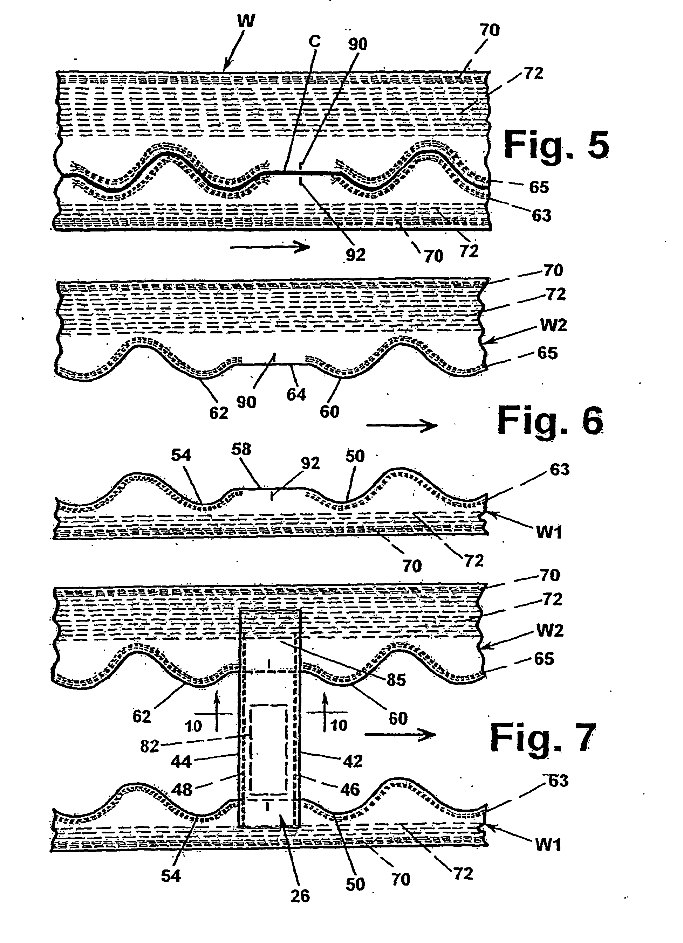

Referring now to Figs. 1, 2 and 10, the details of the construction of the intermediate section 28 will now be discussed. In particular, the section 26 comprises two piles or layers 74 and 78 of the same material as used to form the front and rear sections 22 and 24, respectively. These layers, along with the front and rear sections 22 and 24, respectively, make up the undergarment's chassis. The intermediate section 26 also includes a liquid pervious insert sheet or cover sheet 78, a fluid acquisition layer 80, a liquid absorbent core 82, and a liquid impervious barrier sheet or layer 84. The core 82 is located in a compartment 86 (to be described later) which Is formed between the insert sheet 78 and the inner sheet 74 of the front and rear sections 22 and 24, respectively, as well as certain of the heretofore identified elastic fibers. In particular, as best seen in Fig. 7, the compartment 85 is defined by the intersection of the longitudinally oriented elastic fiber pairs 46 and 48 and the lowermost ones of the transversely oriented elastic fibers 72 of the front belly section and the higher most of ones of the transversely oriented elastic fibers 72 of the rear back section of the chassis. These Intersecting fibers closely surround the core 82 to effectively form a gasket about the periphery of the core to prevent leakage of urine out of the core.

In order to prevent the egress of urine out through the non-woven sheets of the chassis, the intermediate section 26 also includes the heretofore identified barrier sheet or layer 84. The barrier sheet 84 is interposed between the core and the chassis and is formed of a liquid impervious material, e.g., a microporous polyfilm which is vapor permeable (breathable). In the embodiment shown herein, the barrier sheet is of the same generally rectangular shape and size as the cover or insert sheet 78 and is located in the crotch section of the undergarment within the compartment formed by the intersecting elastic threads. In accordance with one exemplary embodiment, the barrier sheet 84 is formed of a film of 0.0127mm (0.5 mil) polypropylene available from Pliant Corporation of Williamsburg, Virginia.

The core 82 is disposed on top of the barrier sheet 84. In the embodiment shown herein, the core 82 is of a generally rectangular shape. If desired, it may have a central portion of increased thickness as compared to its sides marginal portions. The thickened central portion of the core 82 may taper from the front to the rear. These structural features of the core and its location ensures that the maximum thickness of the core is located at the anatomical position at which the urine insult from the wearer will be initiated.

The core 82 can be formed of any suitable material(s), e.g., an airlaid composite, containing pulp, superabsorbent particulates and/or fibers, and binders. The binders may be chemical or thermal.

In accordance with one exemplary embodiment, the core 82 is made up of mainly cellulosic fibers, e.g., wood pulp fluff made of up bleached sulfate wood pulp containing softwood fibers, such as that available from International Paper of Tuxedo, New York, co-mingled with hydrogel polymer particulates (known as Super Absorbent Polymer or "SAP") such as cross-linked polyacrylate IM-3900 available from BASF Corporation of Charlotte, North Carolina. If desired, these materials may be optionally enwrapped in tissue. The amount of each absorbent material and SAP/fluff ratio depends on the size of the protective underwear, whether it is used for children (e.g., training pants) or for adults, and whether or not a transfer or fluid acquisition layer component is to be included in the section 26. In this regard, the fluid acquisition layer 80 as shown herein may be omitted from the undergarment 20, if desired. However, in the embodiment shown the acquisition layer 80 is used between the core 82 and the insert sheet or layer 78. The fluid acquisition layer is located over the thickened portion of the core, i.e., the portion where the urine insult will be initiated. As is known, the fluid acquisition layer 80 serves to manage, transport, accommodate and/or direct high volumes and flow rates of urine into the core.

The fluid acquisition layer 80 can be thru-air bonded/carded web, a spunbond bicomponent non-woven web, a web of crosslink cellulosic fibers, apertured 3D (three dimensional) film or the like. One particularly suitable material is a thru-air bonded bicomponent with a fast finish surfactant available from PGI Non-wovens of Landisville, New Jersey and has an overall basis weight of 40 gsm. The bicomponent fibers are made of a polypropylene inner core and a polyethylene outer sheath. The fluid-acquisition layer 80 may be adhesively secured in place by any suitable construction adhesive, such as 34-373A adhesive available from National Starch and Chemical of Bridgewater, New Jersey. If desired, the core 82 may also be held in place by a similar adhesive.

In accordance with one exemplary embodiment of the undergarment 20, the insert sheet 78 is a non-woven spunbond polypropylene of 15 gsm available from Avgol of Holon, Israel, wherein the non-woven material is zone coated, with its longitudinal central area being hydrophillic and its two longitudinal side areas being hydrophobic. The insert sheet may also be formed of an apertured three dimensional film or a combination of such film and a non-woven material. The insert sheet 78 is of the same size and shape as the barrier sheet 84 and is disposed thereover. The insert sheet 78 is glued to the barrier sheet with the acquisition layer 80, the core 82 and the pairs of elastic fibers 46 and 48 interposed therebetween and completely about the perimeter of the core and acquisition layer. The transversely extending fibers 70 are formed of 620 Decitex LYCRA® and the transversely extending fibers 72 are formed of 800 Decitex LYCRA® available from E.I. DuPont de Nemours of Wilmington, Delaware. The longitudinally extending fibers 46 and 48 are formed of 800 Decitex LYCRA® and fibers 63 and 65 are formed of 940 Decitex LYCRA®.

The use of linearly disposed elastic fibers 46 and 48 in the intermediate section 26 and the arcuately disposed elastic fibers 63 and 65 in the front and rear sections 22 and 24, respectively, cooperate with one another to effectively form a complete peripheral seal to elasticize the leg openings 52 and 56 in lieu of completely encircling the leg openings with an arcuate elastic fiber. As should be appreciated from the discussion to follow, this feature expedites the manufacture of the protective undergarment 20 on a continuous basis. Moreover, by making use of the linearly extending longitudinal elastic fibers 46 and 48 to form a portion of the leg openings, one is able also to use those fibers as a portion of a gasketing system to intersect the transverse fibers 72 to form the heretofore identified sealed compartment 85 to prevent leakage of urine from the core.

It should be pointed out at this juncture that the materials as described heretofore to make up the undergarment 20 are merely exemplary of numerous materials that can be used for the various components. Thus, other conventional materials can be used for the chassis, the insert, its components, and the elastic threads or fibers.

In order to facilitate the correct orientation of the undergarment to put it on, since it is asymmetrical (the rear portion is longer/higher than the front portion), at least some of the elastic fibers of either the front or back of the undergarment may be distinctively colored to be readily discernable from the color of the chassis material to contrast with the color of the chassis, thereby enabling a person to readily determine what is the front and what is the rear of the undergarment so that it can be put on properly.

To put the undergarment on all that is required is for the user to orient the garment in the appropriate direction and to stretch the waist and contiguous upper portion of the chassis to enable his or her leg to be extended through the appropriate leg opening and then through the other leg opening. Then the user can pull the undergarment up so that its waist is located at the wearer's waist and its crotch is located over the wearer's crotch. Thus, the underwear can be put on as easily as any conventional undergarment. Removal of the undergarment is accomplished in a similar manner.

As should be appreciated by those skilled in the art from the foregoing the absorbent articles of this invention are simple in construction, effective and are comfortable to wear.

The undergarments 20 as described above can be made in various ways in accordance with the method(s) of this invention. In fact, one method of this invention can be used to manufacture a disposable diaper which is somewhat similar in construction than the undergarment 20 described above, but whose side marginal edges 32 and 34 of the front and rear sections, 22 and 24, respectively, are not secured together. In such an embodiment securement means, such as adhesive tapes, Velcro® fasteners or any other conventional fasteners found in conventional disposable diapers, can be used.

Referring now to Figs. 3-9, the details of one exemplary method to make absorbent articles, such as the undergarment 20, will now be described. To that end, the two webs of material S1 and S2 are provided from a pair of rolls 102 and 104 and are brought into a confronting relationship with each other. The elastic threads that make up the elastic fibers 63, 65, 70 and 72 are fed from a roll or reel or plural rolls/reels 106 between the webs S1 and S2 and those webs are secured together by an adhesive supplied by applicator 108 to form the unitary web W from which the front and rear sections of the chassis will be formed. The elastic threads forming the waist elastics 70 are fed between the sheets S1 and S2 in a linear fashion so that they extend parallel to each other closely adjacent the side marginal edges of the resulting web W as shown in Fig. 4. The elastic threads forming the belly and back elastics 72 are fed in a similar manner. All of those fibers are fed under tension so that they remain under tension when secured in place.

The fibers forming the elastics for the bottom of the front and rear sections are fed from the roll(s) 106 in an alternating undulating-linear pattern. In particular, as shown in Fig. 4, the threads forming the elastics 63 of the front section's lower edge are provided so that there is an undulating portion 63A followed by a linear portion 63B followed by a mirror image undulating portion 63A and so on. In a similar manner, the threads forming the elastics 65 of the rear section's lower edge are provided so that there is an undulating portion 65A followed by a linear portion 65B followed by a mirror image undulating portion 65A and so on. The linear portions 63B and 65B of the threads 63 and 65 are not adhesively secured to the webs S1 and S2, but are under tension (for reasons to be described later). The two undulating/linear groups of elastic thread lines are disposed parallel to but spaced from each other by a gap G of corresponding shape.

The web W shown in Fig. 4 is fed longitudinally in the direction of the arrow shown therein so that its leading end reaches a slitting station. The slitting station includes a knife or slitter (not shown) for severing the web W into two complementary shaped sections that will become the front section 22 and the rear section 24. To that end, the knife or slitter is moved with respect to the web W while the web is moving in the direction of the arrow so that the knife or slitter follows the path shown by the solid cut line designated by C in Fig. 5. As the web W is slit into the two sections another cutter reciprocates in and out to pierce through the web at 90 and 92 to form slits on opposite sides of the cut line C. This action severs the linearly extending portions 63B and 65B of the threads 63 and 65. Since, as mentioned earlier, those portions of the fibers 63 and 65 are not adhesively secured to the webs S1 and S2, but are under tension, the severed fibers 63 and 65 will snap back toward their contiguous undulating portions which are adhesively secured, leaving the linear portion between the undulating portions empty as shown in Figs. 5 and 6.

The two sections of the web W are then separated from each other to form web sections W1 and W2 as shown in Fig. 6. These sections continue to move in unison in the direction of the arrow to a station for applying the intermediate section 26. At that station a section 26 is brought in and oriented so that its longitudinal axis is perpendicular to the longitudinal axis of the web sections W1 and W2. At this station the section 26 is disposed onto the inner surfaces 74 of the two web sections W1 and W2. An adhesive is applied at this station so that the portion of the section 26 contiguous with its end 38 is adhesively secured to the inner sheet 74 of the web section W1. At the same time and adhesive is applied so that the portion of the section 26 contiguous with its end 40 is adhesively secured to the inner sheet 74 of the web section W2.

Another method of securely attaching the intermediate section 26 onto the inner surfaces 74 of the two web sections W1 and W2 is to ultrasonically bond or heat seal the ends 38 and 40 of section 26 directly to the two web sections W1 and W2. The rest of the intermediate section 26, that is not ultrasonically bonded or heat sealed to web sections W1 and W2, yet in contact with them, can be secured with adhesive.

From this station the two web sections W1 and W2 and the adhesively secured intermediate section 26 move as an assembly in the direction of the arrow to a folding station. At the folding station the intermediate section 26 of the assembled unit is folded along a central line located between the outside marginal edges of the web sections W1 and W2 to bring those sections into a confronting relationship, as shown in Fig. 8.

From there the folded assembly moves to a final sealing and severing station. At that station the confronting web sections W1 and W2 are sealed together at two closely adjacent seal lines 36 on opposite, equidistantly spaced locations with respect to the intermediate section 26, i.e., at locations between the trailing arcuate edges 54 and 62 of the upstream assembly that will become an undergarment 20 and the leading arcuate edges 50 and 60 of the immediately downstream assembly. Once the heat or ultrasonic seals are formed, a severing knife or slitter (not shown) severs the sealed web sections W1 and W2 between the immediately adjacent seal lines 36, to thereby complete an undergarment 20. Each successive assembly is sealed and severed in the same manner to create successive undergarments, so that the undergarments 20 can be produced on a continuous basis.

It should be pointed out that while the method has been described for manufacturing undergarments on a continuous or mass-produced basis, it can be used to continuously mass produce diapers as well. In such an application the side edges of the assembled items, i.e., the items at the stage shown in Fig. 8, are not sealed together, but are left unconnected, and then are severed along their respective sides in a manner like described with respect to Fig. 9. The unsealed sides can then be provided with conventional diaper closures. In particular, fastening tapes or other conventional fasteners can then be applied to the respective sides of the back section of the diaper, to complete it. If the article made is a diaper the transversely extending elastic threads 70 and 72 may be omitted.

As should be appreciated by those skilled in the art, the undergarments 20 or diapers (as the case may be) can be manufactured in accordance with this invention with no wastage of material forming the various sections thereof. In particular, since the front section 22 and rear section 24 are of complementary shape they can be made at one time without any material wastage. Moreover, the elastics can be applied easily, without wastage. Thus, the subject method represents an extremely simple and economical manner of mass producing absorbent undergarments or diapers. Moreover, the undergarments produced exhibit outstanding ergonomic characteristics, without any sacrifice in absorbency and leakage protection. An additional advantage of the manufacturing process of this invention is its extreme flexibility to effect the efficient manufacture different types of absorbent articles without substantial modification to the manufacturing equipment. In particular, the construction of the front and rear sections can be altered as desired to create different undergarments or other similar absorbent articles. Different materials can be used to make the front and rear sections of the undergarment. If the absorbent article is a diaper, different types of fastening tapes (e.g., adhesive, multi-hook, etc.,) can be used, "landing zones" can be incorporated into the front section to facilitate the releasable securement of the fastening tapes thereto and to enable repeated fastening and re-fastening, if desired. Elastic materials can be provided in either or both of the front and rear sections along their top edges in the interests of forming a more secure or closer fit. The shape of the front and rear sections can be readily altered, if desired, by merely altering the pattern of the elastic threads elasticizing the lower edges of the front and rear sections and by correspondingly alternating the shape of the cut line C (although as discussed earlier it is preferable that the lower edge portion of the front panel be concave to result in a high leg opening in the interest of comfort and the lower edge portion of the rear panel be convex to cover the buttocks). So, too, the construction of the intermediate sections can be altered as desired, e.g., different materials can be used for the cover-stock and barrier walls, the core can be of different materials and shapes (e.g., some hour-glass shaped instead of rectangular), a fluid acquisition layer of any particular material or construction may be incorporated into the intermediate section between the cover-stock and the core, etc., standing leg cuffs can be added, etc.

Further economies can be achieved in the manufacturing process by making use of only a single layer or ply of material forming the chassis. Further still, the material making up the barrier wall in the crotch area, if a film, can be of lower gauge in the interest of economics, without the loss of functionality.

Without further elaboration the foregoing will so fully illustrate our invention that others may, by applying current or future knowledge, adopt the same for use under various conditions of service.

## Claims

1. A disposable protective undergarment 20 arranged to be worn by a person having legs to trap and collect loose or liquid waste products of the person, said undergarment comprising a pants-shaped chassis having a front section 22, a rear section 24, a crotch portion 26, and a pair of elasticised leg openings 52, 56 disposed on opposite sides of said crotch portion 26, said front section 22 having a front waist portion and a belly portion 28, said rear section 24 having a rear waist portion and a rear back portion 30, said front waist portion and said rear waist portion being elasticised and joined together at the sides of said chassis 32, 34, said belly portion 28 and said rear back portion 30 being joined together at the sides of said chassis 32, 34, each of said leg openings 52, 56 having an undulating lower edge section at said front section 22 having a high-cut concave edge portion 50, 54, an undulating lower edge section at said rear, section 24 having a convex edge portion 60, 62 and an intermediate edge portion 42, 44 along said crotch portion 26, said concave edge portion 50, 54 of one of said leg openings 52, 56 being of complementary shape to said convex edge portion 60, 62 of said one of said leg openings 52, 56, said concave edge portion 50, 54 of the other of said leg openings 52, 56 being of complementary shape to said convex edge portion 60, 62 of said other of said leg openings 52, 56, whereupon when said undergarment 20 is worn said undergarment 20 covers a portion of the wearers leg beneath the buttocks, while a portion of the wearer's leg where the leg meets the torso is exposed.

2. The disposable protective undergarment as claimed in Claim 1, wherein each of said leg openings 52, 56 is elasticised by at least one elastic thread 63, 65, 46, 48 extending along said concave edge portion 50, 54, said convex edge portion 60, 62 and along said intermediate edge portion 42, 44.

3. The disposable protective undergarment as claimed in any preceding Claim, additionally comprising a plurality of transversely extending elastic threads 70, 72 in said front waist portion, said belly portion 28 and said rear waist portion.

4. The protective undergarment as claimed in any preceding Claim, wherein said crotch portion 26 is a separate member having a pair of sides 42, 44 and a pair of ends 38, 40, one of said ends 38 being secured to said belly portion 28 and the other of said ends 40 being secured to said rear waist portion.

5. The protective undergarment of Claim 4, wherein each of said leg openings 52, 56 is elasticized by at least one elastic thread 63 extending along said concave edge portion 50, 54, by at least one elastic thread 65 extending along said convex edge portion 60,62 and by at least one elastic thread 46, 48 extending along said an associated one of said pair of sides 42, 44 of said crotch portion 26.

6. The protective undergarment as claimed in Claim 4 or Claim 5, wherein said separate member forming said crotch portion 26 is of rectangular shape.

7. The protective undergarment as claimed in any preceding Claim, wherein said chassis comprises a non-woven material,

8. The protective undergarment of Claim 7, wherein said non-woven material comprises spunbond polypropylene.

9. The protective undergarment as claimed in any preceding Claim, additionally comprising a core 82 formed of highly moisture absorbent material.

10. The protective undergarment of Claim 9, additionally comprising a fluid acquisition layer 80 disposed over said core 82.

11. The disposable protective undergarment of Claim3, additionally comprising an absorbent core 82 and an insert sheet 78, and wherein certain ones of said transversely extending elastic threads 70, 72 intersect with said elastic threads 46, 48 of said leg openings 52, 56 to form an enclosed, gasketed compartment 85 for said liquid absorbent core to prevent the egress of liquid therefrom.

12. The protective undergarment of Claim 11, wherein said transversely oriented elastic threads 72 comprise two first groups 70 and two second groups 72, a first one of said two first groups 70 being located in said front waist portion and a second one of said first two groups 70 being located in said rear waist portion, a first one of said two second groups 72 being located in said belly portion 28 and a second one of said two second groups 72 being located in said rear back portion 30, and wherein said transversely oriented threads of said first groups 70 are spaced closer to one another than said transversely oriented threads of said second groups 72.

13. A method for manufacturing plural disposable protective undergarments arranged to be worn by a person having legs to trap and collect loose or liquid waste products of the person, each of said protective undergarments comprising a pants shaped chassis having a front section 22, a rear section 24 and a crotch section 26, said method comprising the steps of:
(A) providing a web of material W having a first longitudinal axis, a pair of linear side edges, a first elongated elastic member 70 extending linearly parallel to said first longitudinal axis adjacent one of said side edges, a second elongated elastic member 70 extending linearly parallel to said first longitudinal axis adjacent the other of said side edges, at least a third undulating curved elastic member 63 extending along the direction of said first longitudinal axis adjacent said first elastic member 70, and at least a fourth undulating curved elastic member 65 extending along the direction of said first longitudinal axis adjacent said second elastic member 70;
(B) severing said web W between said third and fourth elastic members 63, 65 and along the direction of said first longitudinal axis to form a front web section W₁ having undulating lower edge sections defining high-cut concave edge portions 50, 54 and a rear web section W₂ having undulating lower edge sections defining convex edge portions 60, 62;
(C) separating said front and rear web sections from each other;
(D) providing a crotch section 26 having a second longitudinal axis, a pair of marginal side edges 42, 44 extending parallel to said second longitudinal axis, a front edge portion 38, a rear edge portion 40, a first elongated elastic member 46 extending parallel to one of said marginal side edges 42, 44, and a second elongated elastic member 48 extending parallel to the other of said marginal side edges 44, 44;
(E) securing said front edge portion 38 of said crotch section 26 to said front web section W₁ and securing said rear edge portion 38 of said crotch section 26 to said rear web sections W₂ so that said second longitudinal axis is perpendicular to said first longitudinal axis;
(F) severing said front and rear web sections W₁, W₂ along respective transverse lines extending perpendicular to said first longitudinal axis on opposite sides of said crotch section 26.

14. The method of Claim 13, wherein said method additionally comprises the step of:
(G) folding said crotch section 26 to bring portions of said front and rear sections W₁, W₂ contiguous with said transverse lines into engagement with each other and securing said sections together thereat, so that said severed front web section W₁ forms a front portion 22 of said undergarment and said severed rear web section W₂ forms a rear portion 24 of said undergarment.

15. The method of Claim 14, wherein said method additionally comprises:
(H) providing a pair of web layers S₁, S₂, each formed of a flexible material; and
(I) interposing said elastic members 63, 65, 70 between said web layers S₁, **S₂** and laminating said layers together to form said web of material W.

16. The method as claimed in any one of Claims 13 to 15, wherein said elongated elastic members 63, 65, 70 are provided in the form of elastic threads.

17. The method of Claim 14, wherein said third elastic member 63 is one of a third group of plural elastic threads extending along the direction of said first longitudinal axis, and said fourth elastic member 65 is one of a fourth group of plural elastic threads extending along the direction of said first longitudinal axis.

18. The method of Claim 17, wherein said third group of elastic threads 63 are disposed in discontinuous segments spaced from each other along the direction of said first longitudinal axis, and wherein said fourth group of elastic threads 65 are disposed in discontinuous segments spaced from each other along the direction of said first longitudinal axis.

19. The method as claimed in Claim 17 or 18, wherein said method additionally comprises:
(H) providing each of said threads of said third group of elastic threads 63 as a continuous member having undulating portions 63A and linear portions 63B interposed with each other and wherein each of said continuous members is under tension, with said undulating portions 63A of each of said continuous threads being adhesively secured to said web of material W and said linear portion 63B of each of said continuous threads being unsecured to said web W;
(I) providing each of said threads of said fourth group of elastic threads 65 as a continuous member having undulating portions 65A and linear portions 65B interposed with each other and wherein each of said continuous members is under tension, with said undulating portions 65A of each of said continuous threads being adhesively secured to said web of material W and said linear portion 65B of each of said continuous threads being unsecured to said web W; and
(J) severing each of said threads of said third and fourth groups of elastic threads 63, 65 at said linear portions 63B, 65B to cause said each of said threads of said third and fourth groups of elastic threads 63, 65 to be disposed in discontinuous segments spaced from each other in a direction parallel to said first longitudinal axis.

20. The method as claimed in any one of Claims 13 to 19. wherein said first elongated elastic member 70 is one of a first group of plural elastic threads extending continuously linearly parallel to said first longitudinal axis adjacent one of said side edges, said second elongated elastic member 70 is one of a second group of plural elastic threads extending continuously linearly parallel to said first longitudinal axis adjacent the one of said side edges.

21. The method as claimed in any one of Claims 13 to 20, wherein said web of material W is moved in a direction parallel to said first longitudinal axis.

22. The method of Claim 21, wherein said web w is severed along said longitudinal line between said third and fourth elastic members 63, 65 as it is moving.

23. The method as claimed in Claim 21 or 22, wherein said crotch 26 sections are provided at spaced apart intervals on said front and rear web sections W₁, W₂ as said front and rear web sections W₁, W₂ are moving in a direction parallel to said first longitudinal axis.

24. The method as claimed in any one of Claim 21 to 23, wherein said crotch sections 26 are secured to said front and rear web sections W₁, W₂ as said web W is moving.

25. The method as claimed in Claim 24, wherein the folding of said crotch section 26, the securing of said front and rear web sections W₁, W₂ together and the severing of said sections W₁, W₂ thereat is accomplished as said web W is moving.

## Patentansprüche

1. Wegwerfbare Schutzunterwäsche (20), die von einer Person mit Beinen getragen werden kann, um lose oder flüssige Abfallprodukte von der Person aufzunehmen und zu sammeln, wobei die Unterwäsche ein als Hose geformtes Grundgerüst aufweist, das einen vorderen Abschnitt (22), einen rückwärtigen Abschnitt (24), einen Schrittbereich (26) und ein Paar von elastischen Beinöffnungen (52, 56) aufweist, die auf gegenüberliegenden Seiten des Schrittbereichs (26) ausgebildet sind, wobei der vordere Abschnitt (22) einen vorderen Taillenbereich und einen Bauchbereich (28) enthält, wobei der rückwärtige Abschnitt (24) einen rückwärtigen Taillenbereich und einen rückwärtigen hinteren Bereich (30) enthält, wobei der vordere Taillenbereich und der rückwärtige Taillenbereich elastisch ausgebildet sind und an den Seiten des Grundgerüsts (32, 34) verbunden sind, wobei der Bauchbereich (28) und der rückwärtige hintere Bereich (30) an den Seiten des Grundgerüsts (32, 34) verbunden sind, und jede Beinöffnung (52, 56) einen wellenförmigen unteren Kantenabschnitt an dem vorderen Abschnitt (22) aufweist, der einen hochgeschnittenen konkaven Kantenbereich (50, 54) aufweist, wobei ein wellenförmiger unterer Kantenabschnitt an dem rückwärtigen Abschnitt (24) einen konvexen Kantenabschnitt (60, 62) und einen mittleren Kantenabschnitt (42, 44) entlang dem Schrittbereich (26) enthält, wobei der konkave Kantenbereich (50, 54) an einer der Beinöffnungen (52, 56) eine komplementäre Form zum konvexen Kantenabschnitt (60, 62) des einen der Beinöffnungen (52, 56) aufweist, wobei der konkave Kantenabschnitt (50, 54) des anderen der Beinöffnungen (52, 56) eine komplementäre Form zum konvexen Kantenabschnitt (60, 62) des anderen der Beinöffnungen (52, 56) hat, wodurch beim Tragen der Unterwäsche (20) die Unterwäsche (20) einen Teil des Beins des Trägers unterhalb des Gesäßes abdeckt, während ein Teil des Beins des Trägers, wo das Bein den Körper trifft, freiliegt.

2. Wegwerfbare Schutzunterwäsche nach Anspruch 1, bei der jede der Beinöffnungen (52, 56) durch wenigstens einen elastischen Faden (63, 65, 46, 48), der sich entlang dem konkaven Kantenbereich (50, 54), dem konvexen Kantenbereich (60, 62) und entlang dem mittleren Kantenbereich (42, 44) erstreckt, elastisch ausgebildet ist.

3. Wegwerfbare Schutzunterwäsche nach einem der vorhergehenden Ansprüche, welche zusätzlich eine Mehrzahl von querverlaufenden elastischen Fäden (70, 72) in dem vorderen Taillenbereich, dem Bauchbereich (28) und dem rückwärtigen Taillenbereich aufweist.

4. Schutzunterwäsche nach einem der vorhergehenden Ansprüche, bei dem der Schrittbereich (26) ein separates Teil ist, das ein Paar von Seiten (42, 44) und ein Paar von Enden (38, 40) aufweist, wobei eines der Enden (38) an dem Bauchbereich (28) befestigt ist und das andere der Enden (40) mit dem rückwärtigen Taillenbereich verbunden ist.

5. Schutzunterwäsche nach Anspruch 4, bei der jede der Beinöffnungen (52, 56) durch wenigstens einen elastischen Faden (63) elastisch ist, der sich entlang dem konkaven Kantenbereich (50, 54) durch wenigstens einen elastischen Faden (65) erstreckt, der entlang dem konvexen Kantenbereich (60, 62) verläuft und durch wenigstens einen elastischen Faden (46, 48), der sich entlang einer zugeordneten Seite des Paares von Seiten (42, 44) des Schrittbereichs (26) erstreckt.

6. Schutzunterwäsche nach Anspruch 4 oder 5, bei der das getrennte Teil, das den Schrittbereich (26) bildet, eine rechteckige Form aufweist.

7. Schutzunterwäsche nach einem der vorhergehenden Ansprüche, bei der das Grundgerüst nicht-gewebtes Material enthält.

8. Schutzunterwäsche nach Anspruch 7, bei der das nicht-gewebte Material gesponnenes Polypropylen enthält.

9. Schutzunterwäsche nach einem der vorhergehenden Ansprüche, welche zusätzlich einen Kern (82) aufweist, der aus stark feuchtigkeitsabsorbierendem Material besteht.

10. Schutzunterwäsche nach Anspruch 9, welche eine zusätzliche flüssigkeitssammelnde Schicht (80) über dem Kern (82) aufweist.

11. Wegwerfbare Schutzunterwäsche nach Anspruch 3, welche zusätzlich einen absorbierenden Kern (82) und eine Einlage (78) enthält, und wobei bestimmte der querverlaufenden elastischen Fäden (70, 72) die elastischen Fäden (46, 48) der Beinöffnungen (52, 56) kreuzen, um einen geschlossenen korbartigen Aufnahmeraum (85) für den flüssigkeitsabsorbierenden Kern zu erhalten, um den Ausfluss von Flüssigkeit daraus zu verhindern.

12. Schutzunterwäsche nach Anspruch 11, bei der die querverlaufenden elastischen Fäden (72) wenigstens zwei erste Gruppen (70) und zwei zweite Gruppen (72) enthalten, wobei eine erste der zwei ersten Gruppen (70) im vorderen Taillenbereich und eine zweite der ersten zweiten Gruppen (70) im hinteren Taillenbereich angeordnet sind, eine erste der zwei zweiten Gruppen (72) im Bauchbereich (28) und eine zweite der zwei zweiten Gruppen (72) im hinteren rückwärtigen Bereich (30) ausgebildet ist, und wobei die querverlaufenden Fäden der ersten Gruppen (70) näher zueinander als die querverlaufenden Bänder der zweiten Gruppen (72) beabstandet sind.

13. Verfahren zur Herstellung mehrerer wegwerfbarer Schutzunterwäschestücke, die durch eine Person mit Beinen getragen werden kann, um lose oder flüssige Abfallprodukte der Person aufzunehmen und zusammeln, wobei jede der Schutzunterwäschestücke ein hosenförmig geformtes Grundgerüst aufweist, das einen vorderen Abschnitt (22), einen rückwärtigen Abschnitt (24) und einen Schrittabschnitt (26) enthält, wobei das Verfahren die folgenden Schritte aufweist:
(A) Vorsehen einer Materialbahn W, das eine erste Längsachse, ein Paar von linearen Seitenkanten, ein erstes längliches elastisches Teil (70), das sich linear parallel zu der ersten Längsachse nahe einer der Seitenkanten erstreckt, ein zweites längliches elastisches Teil (70), das linear parallel zur ersten Längsachse neben der anderen der Seitenkanten verläuft, wenigstens ein drittes wellenförmig geformtes elastisches Glied (63), das sich entlang der Richtung der ersten Längsachse neben dem ersten elastischen Teil (70) erstreckt und wenigstens ein viertes wellenförmig geformtes elastisches Teil (65), das sich entlang der Richtung der ersten Längsachse an dem zweiten elastischen Teil (70) erstreckt, aufweist,
(B) Teilen der Bahn W zwischen dem ersten und vierten elastischen Teil (63, 65) und entlang der Richtung der ersten Längsachse, um einen vorderen Bahnabschnitt W₁ mit einem unteren wellenförmigen Kantenabschnitt, welcher hochgeschnittene konkave Kantenbereiche (50, 54) aufweist, und einem rückwärtigen Bahnabschnitt W₂, welcher wellenförmige untere Kantenabschnitte aufweist, die konvexe Kantenbereiche (60, 62) bilden, zu formen,
(C) Trennung der vorderen und hinteren Bahnabschnitte voneinander,
(D) Vorsehen eines Schrittabschnitts (26) mit einer zweiten Längsachse, einem Paar von randseitigen Kanten (42, 44), die sich parallel zur zweiten Längsachse erstrecken, einem vorderen Kantenbereich (38), einem rückwärtigen Kantenbereich (40), einem ersten elastischen Teil (46), das sich parallel zu einem der randseitigen Kanten (42, 44) erstreckt, und einem zweiten elastischen Element (48), das sich parallel zu dem anderen der randseitigen Kanten (44, 44) erstreckt,
(E) Befestigen des vorderen Kantenbereichs (38) des Schrittabschnitts (26) an dem vorderen Bahnabschnitt W₁ und Befestigen des rückwärtigen Kantenbereichs (38) des Schrittabschnitts (26) an dem rückwärtigen Bahnabschnitt W₂, so dass die zweite Längsachse rechtwinklig zur ersten Längsachse verläuft,
(F) Trennung der vorderen und rückwärtigen Bahnabschnitte W₁, W₂ entlang jeweilig querverlaufender Linien, die sich rechtwinklig zur ersten Längsachse auf gegenüberliegenden Seiten des Schrittabschnitts (26) erstrecken.

14. Verfahren nach Anspruch 13, welches zusätzlich die folgenden Schritte enthält:
(G) Falten des Schrittabschnitts (26), um Teile der vorderen und hinteren Abschnitte W₁, W₂ benachbart zu den querverlaufenden Linien miteinander auszurichten und daraufhin Befestigen der Abschnitte zusammen der abgetrennte vordere Bahnabschnitt W₁ einen vorderen Bereich (22) der Unterwäsche bildet und der abgetrennte rückwärtige Bahnenabschnitt W₂ einen rückwärtigen Bereich (24) der Unterwäsche bildet.

15. Verfahren nach Anspruch 14, wobei das Verfahren zusätzlich enthält:
(H) Vorsehen eines Paars von Bahnschichten S₁, S₂, die jeweils aus einem flexiblen Material bestehen, und
(I) Einfügen der elastischen Elemente (63, 65, 70) zwischen die Bahnschichten S₁, S₂ und Laminieren der Schichten zusammen, um das Bahnmaterial W zu bilden.

16. Verfahren nach einem der Ansprüche 13 - 15, bei dem die gestreckten elastischen Elemente (63, 65, 70) in Form von elastischen Bändern ausgebildet sind.

17. Verfahren nach Anspruch 14, bei dem das dritte elastische Teil (63) eines der dritten Gruppe von mehreren elastischen Bändern ist, die sich entlang der Richtung der ersten Längsachse erstrecken, und das vierte elastische Teil (65) eines einer vierten Gruppe von mehreren elastischen Bändern ist, die sich entlang der Richtung der ersten Längsachse erstrecken.

18. Verfahren nach Anspruch 17, bei dem die dritte Gruppe der elastischen Fäden (63) in nicht kontinuierlichen Segmenten angeordnet ist, die voneinander entlang der Richtung der ersten Längsachse beabstandet sind, und wobei die vierte Gruppe von elastischen Fäden (65) in nicht kontinuierlichen Segmenten angeordnet sind, die voneinander in Richtung der ersten Längsachse beabstandet sind.

19. Verfahren nach Anspruch 17 oder 18, bei dem das Verfahren zusätzlich enthält:
(H) Vorsehen der Fäden der dritten Gruppe von elastischen Fäden (63) als kontinuierliches Element mit wellenförmigen Bereichen (63A) und linearen Bereichen (63B), die miteinander verschränkt sind, und wobei jede der kontinuierlichen Elemente unter Spannung steht, wobei die wellenförmigen Bereiche (63A) jeder der kontinuierlichen Fäden durch Klebung an dem Bahnmaterial W befestigt sind, und der lineare Bereich (63B) jedes der kontinuierlichen Fäden nicht an dem Bahnbereich W befestigt ist,
(I) Vorsehen jedes der Fäden der vierten Gruppe von elastischen Fäden (65) als kontinuierliches Element mit wellenförmigen Bereichen (65A) und linearen Bereichen (65B), die miteinander verschränkt sind, und wobei jedes der kontinuierlichen Elemente unter Spannung steht, wobei die wellenförmigen Bereiche (65A) jedes der kontinuierlichen Fäden durch Klebung an dem Bahnmaterial W befestigt ist, und der lineare Bereich (65B) jedes der kontinuierlichen Fäden nicht an der Bahn W befestigt ist, und
(J) Trennung jedes der Fäden der dritten und vierten Gruppen von elastischen Fäden (63, 65) an den linearen Bereichen (63B, 65B), um jeden der Fäden der dritten und vierten Gruppe der elastischen Fäden (63, 65) als nicht kontinuierliche Segmente auszubilden, die voneinander in einer Richtung parallel zu der ersten Längsachse beabstandet sind.

20. Verfahren nach einem der Ansprüche 13 - 19, bei dem das erste elastische Längsteil (70) eines der ersten Gruppe von mehreren elastischen Fäden ist, die sich kontinuierlich parallel zur ersten Längsachse neben einer der Seitenkanten erstrecken, wobei das zweite elastische Längselement (70) eines einer zweiten Gruppe von mehreren elastischen Fäden ist, die sich kontinuierlich linear parallel zu der ersten Längsachse neben einer der Seitenkanten erstrecken.

21. Verfahren nach einem der Ansprüche 13-20, wobei das Bahnmaterial W in einer Richtung parallel zur ersten Längsachse bewegt wird.

22. Verfahren nach Anspruch 21, wobei die Bahn W entlang der Längslinie zwischen den ersten und vierten elastischen Elementen (63, 65) getrennt wird, während sie sich bewegt.

23. Verfahren nach Anspruch 21 oder 22, bei dem die Schrittabschnitte (26) in voneinander beabstandeten Intervallen an den vorderen- und rückwärtigen Bahnabschnitten W₁, W₂ vorgesehen sind, während die vorderen und rückwärtigen Bahnabschnitte W₁, W₂ in einer Richtung parallel zur ersten Längsachse bewegt werden.

24. Verfahren nach einem der Ansprüche 21 - 23, wobei die Schrittabschnitte (26) mit den vorderen und rückwärtigen Bahnabschnitten W₁, W₂ verbunden werden, während die Bahn W sich bewegt.

25. Verfahren nach Anspruch 24, bei dem das Falten des Schrittabschnitts (26), das Befestigen des vorderen und rückwärtigen Bahnabschnitte W₁, W₂ zusammen und das Trennen der Abschnitte W₁, W₂ durchgeführt wird, während die Bahn W bewegt wird.

## Revendications

1. Sous-vêtement protecteur jetable 20 agencé pour être porté par une personne ayant des jambes pour piéger et recueillir des déchets mous ou liquides de la personne, ledit sous-vêtement comprenant une structure en forme de caleçon ayant une section avant 22, une section arrière 24, une portion d'entrejambe 26 et deux ouvertures de jambes élastiquées 52, 56, disposées sur les côtés opposés de ladite portion d'entrejambe 26, ladite section avant 22 ayant une portion de taille avant et une portion de ventre 28, ladite section arrière 24 ayant une portion de taille arrière et une portion de dos arrière 30, ladite portion de taille avant et ladite portion de taille arrière étant élastiquées et assemblées conjointement sur les côtés de ladite structure 32, 34, ladite portion de ventre 28 et ladite portion de dos arrière 30 étant assemblées ensemble sur les côtés de ladite structure 32, 34, chacune desdites ouvertures de jambes 52, 56 ayant une section de bord inférieure ondulée sur ladite section avant 22 ayant une portion de bord concave de haute coupe 50, 54, une section de bord inférieure ondulée sur ladite section arrière 24 ayant une portion de bord convexe 60, 62 et une portion de bord intermédiaire 42, 44 le long de ladite portion d'entrejambe 26, ladite portion de bord concave 50, 54 d'une desdites ouvertures de jambes 52, 56 étant de forme complémentaire avec ladite portion de bord convexe 60, 62 de ladite une desdites ouvertures de jambes 52, 56, ladite portion de bord concave 50, 54 de l'autre desdites ouvertures de jambes 52, 56 étant de forme complémentaires avec ladite portion de bord convexe 60, 62 de ladite autre desdites ouvertures de jambes 52, 56, moyennant quoi, lorsque ledit sous-vêtement 20 est porté, ledit sous-vêtement 20 recouvre une portion de la jambe de l'utilisateur en dessous des fesses, tandis qu'une portion de la jambe de l'utilisateur où la jambe rejoint le torse est exposée.

2. Sous-vêtement protecteur jetable selon la revendication 1, dans lequel chacune desdites ouvertures de jambes 52, 56 est élastiquée par au moins un fil élastique 63, 65, 46, 48 s'étendant le long de ladite portion de bord concave 50, 54, de ladite portion de bord convexe 60, 62 et de ladite portion de bord intermédiaire 42, 44.

3. Sous-vêtement protecteur jetable selon l'une quelconque des revendications précédentes, comprenant une pluralité de fils élastiques 70, 72 s'étendant transversalement dans ladite portion de taille avant, ladite portion de ventre 28 et ladite portion de taille arrière.

4. Sous-vêtement protecteur selon l'une quelconque des revendications précédentes, dans lequel ladite portion d'entrejambe 26 est un élément séparé ayant une paire de côtés 42, 44 et une paire d'extrémités 38, 40, une desdites extrémités 38 étant fixée à ladite portion de ventre 28 et l'autre desdites extrémités 40 étant fixée à ladite portion de taille arrière.

5. Sous-vêtement protecteur selon la revendication 4, dans lequel chacune desdites ouvertures de jambes 52, 56 est élastiquée par au moins un fil élastique 63 s'étendant le long de ladite portion de bord concave 50, 54, par au moins un fil élastique 65 s'étendant le long de ladite portion de bord convexe 60, 62 et par au moins un fil élastique 46, 48 s'étendant le long dudit un côté associé de ladite paire de côtés 42, 44 de ladite portion d'entrejambe 26.

6. Sous-vêtement protecteur selon la revendication 4 ou la revendication 5, dans lequel ledit élément séparé formant ladite portion d'entrejambe 26 est de forme rectangulaire.

7. Sous-vêtement protecteur selon l'une quelconque des revendications précédentes, dans lequel ladite structure comprend un matériau non tissé.

8. Sous-vêtement protecteur selon la revendication 7, dans lequel ledit matériau non tissé comprend du polypropylène lié au filé.

9. Sous-vêtement protecteur selon l'une quelconque des revendications précédentes, comprenant de plus une âme 82 formée de matériau absorbant fortement l'humidité.

10. Sous-vêtement protecteur selon la revendication 9, comprenant de plus une couche d'acquisition de fluide 80 disposée sur ladite âme 82.

11. Sous-vêtement protecteur jetable selon la revendication 3, comprenant de plus une âme absorbante 82 et une feuille rapportée 78, et dans lequel certains desdits fils élastiques s'étendant transversalement 70, 72 coupent lesdits fils élastiques 46, 48 desdites ouvertures de jambes 52, 56 pour former un compartiment fermé étanche 85 pour ladite âme absorbant les liquides afin d'empêcher le liquide d'en sortir.

12. Sous-vêtement protecteur selon la revendication 11, dans lequel lesdits fils élastique orientés transversalement 72 comprennent deux premier groupes 70 et deux deuxièmes groupes 72, un premier desdits deux premiers groupes 70 étant placé dans ladite portion de taille avant et un second desdits deux premiers groupes 70 étant placé dans ladite portion de taille arrière, un premier desdits deux deuxièmes groupes 72 étant placé dans ladite portion de ventre 28 et un second desdits deux deuxièmes groupes 72 étant placé dans ladite portion de dos arrière 30, et dans lequel lesdits fils orientés transversalement desdits premiers groupes 70 sont espacés plus près l'un de l'autre que lesdits fils orientés transversalement desdits deuxièmes groupes 72.

13. Procédé de fabrication de plusieurs sous-vêtements protecteurs jetables aménagés pour être portés par une personne ayant des jambes pour piéger et recueillir des déchets mous ou liquides de la personne, chacun desdits sous-vêtements protecteurs comprenant une structure en forme de caleçon ayant une section avant 22, une section arrière 24 et une section d'entrejambe 26, ledit procédé comprenant les étapes consistant à :
(A) mettre en oeuvre une nappe de matériau W ayant un premier axe longitudinal, une paire de bords latéraux linéaires, un premier élément élastique allongé 70 s'étendant de manière linéaire parallèlement audit premier axe longitudinal adjacent à l'un desdits bords latéraux, un deuxième élément élastique allongé 70 s'étendant de manière linéaire parallèlement audit premier axe longitudinal adjacent à l'autre desdits bords latéraux, au moins un troisième élément élastique incurvé ondulé 63 s'étendant dans la direction dudit premier axe longitudinal adjacent audit premier élément élastique 70, et au moins un quatrième élément élastique incurvé ondulé 65 s'étendant dans la direction dudit premier axe longitudinal adjacent audit second élément élastique 70 ;
(B) sectionner ladite nappe W entre lesdits troisième et quatrième éléments élastiques 63, 65 et dans la direction dudit premier axe longitudinal pour former une section de nappe avant W1 ayant des sections de bord inférieures ondulées définissant des portions de bord concaves de haute coupe 50, 54 et une section de nappe arrière W2 ayant des sections de bord inférieures ondulées définissant des portions de bord convexes 60, 62 ;
(C) séparer lesdites sections de nappe avant et arrière l'une de l'autre ;
(D) former une section d'entrejambe 26 ayant un second axe longitudinal, une paire de bords latéraux marginaux 42, 44 s'étendant parallèlement audit second axe longitudinal, une portion de bord avant 38, une portion de bord arrière 40, un premier élément élastique allongé 46 s'étendant parallèlement à l'un desdits bords latéraux marginaux 42, 44, et un deuxième élément élastique allongé 48 s'étendant parallèlement à l'autre desdits bords latéraux marginaux 44, 44 ;
(E) fixer ladite portion de bord avant 38 de ladite section d'entrejambe 26 à ladite section de nappe avant W1 et fixer ladite portion de bord arrière 38 de ladite section d'entrejambe 26 auxdits sections de nappe arrière W2 de sorte que ledit second axe longitudinal soit perpendiculaire audit premier axe longitudinal ;
(F) sectionner lesdites sections de nappe avant et arrière W1, W2 le long de lignes transversales respectives s'étendant perpendiculairement audit premier axe longitudinal sur des côtés opposés de ladite section d'entrejambe 26.

14. Procédé selon la revendication 13, dans lequel ledit procédé comprend de plus l'étape consistant à :
(G) plier ladite section d'entrejambe 26 pour amener des portions desdites sections avant et arrière W1, W2 contigües auxdites lignes transversales en engagement l'une avec l'autre et fixer lesdites sections ensemble à cet emplacement, de sorte que ladite section de nappe avant sectionnée W1 forme une portion avant 22 dudit sous-vêtement et que ladite section de nappe arrière sectionnée W2 forme une portion arrière 24 dudit sous-vêtement.

15. Procédé selon la revendication 14, dans lequel ledit procédé comprend de plus les étapes consistant à :
(H) mettre en oeuvre une paire de couches de nappe S1, S2, formées chacune d'un matériau flexible ; et
(I) interposer lesdits éléments élastiques 63, 65, 70 entre lesdites couches de nappe S1, S2 et appliquer lesdites couches l'une sur l'autre pour former ladite nappe de matériau W.

16. Procédé selon l'une quelconque des revendications 13 à 15, dans lequel lesdits éléments élastiques allongés 63, 65, 70 sont façonnés sous la forme de fils élastiques.

17. Procédé selon la revendication 14, dans lequel ledit troisième élément élastique 63 est un fil d'un troisième groupe de plusieurs fils élastiques s'étendant dans la direction dudit premier axe longitudinal, et ledit quatrième élément élastique 65 est un fil d'un quatrième groupe de plusieurs fils élastiques s'étendant dans la direction dudit premier axe longitudinal.

18. Procédé selon la revendication 17, dans lequel les fils dudit troisième groupe de fils élastiques 63 sont disposés en segments discontinus espacés l'un de l'autre dans la direction dudit premier axe longitudinal et dans lequel les fils dudit quatrième groupe de fils élastiques 65 sont disposés en segments discontinus espacés l'un de l'autre dans la direction dudit quatrième axe longitudinal.

19. Procédé selon la revendication 17 ou 18, dans lequel ledit procédé comprend de plus les étapes consistant à :
(H) mettre en oeuvre chacun desdits fils dudit troisième groupe de fils élastiques 63 sous la forme d'un élément continu ayant des portions ondulées 63A et des portions linéaires 63B interposées l'une par l'autre et dans lequel chacun desdits éléments continus est sous tension, lesdites portions ondulées 63A de chacun desdits fils continus étant fixées par un adhésif à ladite nappe de matériau W et ladite portion linéaire 63B de chacun desdits fils continus n'étant pas fixée à ladite nappe W ;
(I) mettre en oeuvre chacun desdits fils dudit quatrième groupe de fils élastiques 65 sous la forme d'un élément continu ayant des portions ondulées 65A et des portions linéaires 65B interposées l'une par l'autre et dans lequel chacun desdits éléments continus est sous tension, lesdites portions ondulées 65A de chacun desdits fils continus étant fixées par un adhésif à ladite nappe de matériau W et ladite portion linéaire 65B de chacun desdits fils continus n'étant pas fixée à ladite nappe W ; et
(J) sectionner chacun desdits fils desdits troisième et quatrième groupes de fils élastiques 63, 65 sur lesdites portions linéaires 63B, 65B pour faire en sorte que chacun desdits fils desdits troisième et quatrième groupes de fils élastiques 63, 65 soit disposé en segments discontinus espacés l'un de l'autre dans une direction parallèle audit premier axe longitudinal.

20. Procédé selon l'une quelconque des revendications 13 à 19, dans lequel ledit premier élément élastique allongé 70 est un fil d'un premier groupe de plusieurs fils élastiques s'étendant de manière linéaire et continue, parallèlement audit premier axe longitudinal adjacent à l'un desdits bords latéraux, ledit deuxième élément élastique allongé 70 est un fil d'un deuxième groupe de plusieurs fils élastiques s'étendant de manière linéaire et continue, parallèlement audit premier axe longitudinal adjacent à l'un desdits bords latéraux.

21. Procédé selon l'une quelconque des revendications 13 à 20, dans lequel ladite nappe de matériau W est déplacée dans une direction parallèle audit premier axe longitudinal.

22. Procédé selon la revendication 21, dans lequel ladite nappe W est sectionnée le long de ladite ligne longitudinale entre lesdits troisième et quatrième éléments élastiques 63, 65 à mesure qu'elle se déplace.

23. Procédé selon la revendication 21 ou 22, dans lequel lesdites sections d'entrejambe 26 sont placées à intervalles espacés l'une de l'autre sur lesdites sections de nappe avant et arrière W1, W2 lorsque lesdites sections de nappe avant et arrière W1, W2 se déplacent dans une direction parallèle audit premier axe longitudinal.

24. Procédé selon l'une quelconque des revendications 21 à 23, dans lequel lesdites sections d'entrejambe 26 sont fixées auxdites sections de nappe avant et arrière W1, W2 à mesure que ladite nappe W se déplace.

25. Procédé selon la revendication 24, dans lequel le pliage de ladite section d'entrejambe 26, la fixation desdites sections avant et arrière W1, W2 ensemble et le sectionnement desdites sections W1, W2 à cet emplacement sont effectués à mesure que ladite nappe W se déplace.
